# EUROPEAN PATENT APPLICATION

(11) **EP 2 332 612 A1**
(43) Date of publication of application: **15.06.2011**
(21) Application number: 09817830.4
(22) Date of filing: 30.09.2009
(51) Int. Cl.: A61N 5/04

(54) **METHOD FOR REDUCING DEGREE OF ACTIVATION OF CELLS, AND APPARATUS FOR THE METHOD**

(30) Priority: 30.09.2008 JP 2008255414
(71) Applicant: Panasonic Electric Works Co., Ltd., Kadoma-shi Osaka 571-8686 (JP)
(72) Inventor: NAKAGAWA, Takehiro, Kadoma-shi Osaka 571-8686 (JP); HAMADA, Chosei, Kadoma-shi Osaka 571-8686 (JP)
(74) Representative: Appelt, Christian W.
(86) International application number: PCT/JP2009/067070
(87) International publication number: WO 2010/038798

(57) **Abstract**

The activity of cells is reduced without administering drugs or performing surgical removals. Electromagnetic waves having a frequency of 30 GHz to 3 THz are emitted to cells to reduce activity of the cells.

## Description

### TECHNICAL FIELD

The present invention relates to a method and apparatus for reducing the activity of cells.

### BACKGROUNDART

A vasoconstrictor is known to suppress cell activity. Japanese Laid-Open Patent Publication No. 2001-220355 describes a cell mass activation suppressing drug.

It is most desirable that the activity of cancer cells be reduced. The removal of cancer cells requires high-skill medical techniques and is highly risky. Moreover, when cancer cells are spread over a wide area, further accurate surgical treatment is required, and risks become even higher. Further, the administration of anticancer drugs may produce side effects such as vomiting, strong fatigue, lassitude, and hair loss. This may result in the patient having to experience unbearable discomfort.

Hence, there is a strong demand for a method and apparatus that reduces the activity of cell to stop or suspend the activity of cancer cells.

### PRIOR ART DOCUMENT

Patent Document 1: Japanese Laid-Open Patent Publication No. 2001-220355

### SUMMARY OF THE INVENTION

### PROBLEMS THAT ARE TO BE SOLVED BY THE INVENTION

Accordingly, the present invention provides a method and apparatus for reducing the activity of cells without administering drugs or performing surgical removals.

### MEANS FOR SOLVING THE PROBLEM

To solve the above problem, a cell activity reducing method according to the present invention emits electromagnetic waves having a frequency of 30 GHz to 3 THz to cells to reduce activity of the cells. A cell activity reduction apparatus according to the present invention includes an electromagnetic wave emission means for emitting electromagnetic waves having a frequency of 30 GHz to 3 THz that reduces cell activity.

The emission of millimeter waves and terahertz waves in the frequency band of 30 GHz to 3 THz to target cells reduces the activity of the cells. There is no need to perform surgical treatment depending on the portion, such as skin surface, throat, nasal cavity, and organ surface. This lowers risks and greatly reduces the discomfort experienced by the patient. Further, side effects caused by the use of drugs are avoided.

An experiment for proving the cell activity reduction effect of electromagnetic waves preferably includes an electric resistance measurement means for measuring an electric resistance of the cells, which is an irradiation subject of electromagnetic waves, or a culture liquid for the cells, which is an irradiation subject of electromagnetic waves, and a display means for displaying the electric resistance value measured by the electric resistance measurement means or a pH measurement means for measuring the pH of a culture liquid for cells, which is an irradiation subject of electromagnetic waves, and a display means for displaying the pH value measured by the pH measurement means.

### EFFECT OF THE INVENTION

The present invention reduces the activity of cells by emitting electromagnetic waves having a frequency of 30 GHz to 3 THz. This safely reduces the activity of cells without causing side effects.

### BRIEF DESCRIPTION OF THE DRAWINGS

Fig. 1 is a block diagram of an electromagnetic wave emission unit according to one embodiment of the present invention;
Fig. 2 is a block diagram showing the entire structure of a cell holding unit of Fig. 1;
Fig. 3 is a block diagram showing an electric resistance measurement unit of Fig. 1;
Fig. 4A is a side view showing another example of the cell holding unit, and Fig. 4B is a plan view showing a further example of the cell holding unit; and
Fig. 5A is a side view and Fig. 5B is a plan view showing another example of the cell holding unit; and
Fig. 6 is a chart showing the cell activity reduction effect of electromagnetic waves.

### EMBODIMENT OF THE INVENTION

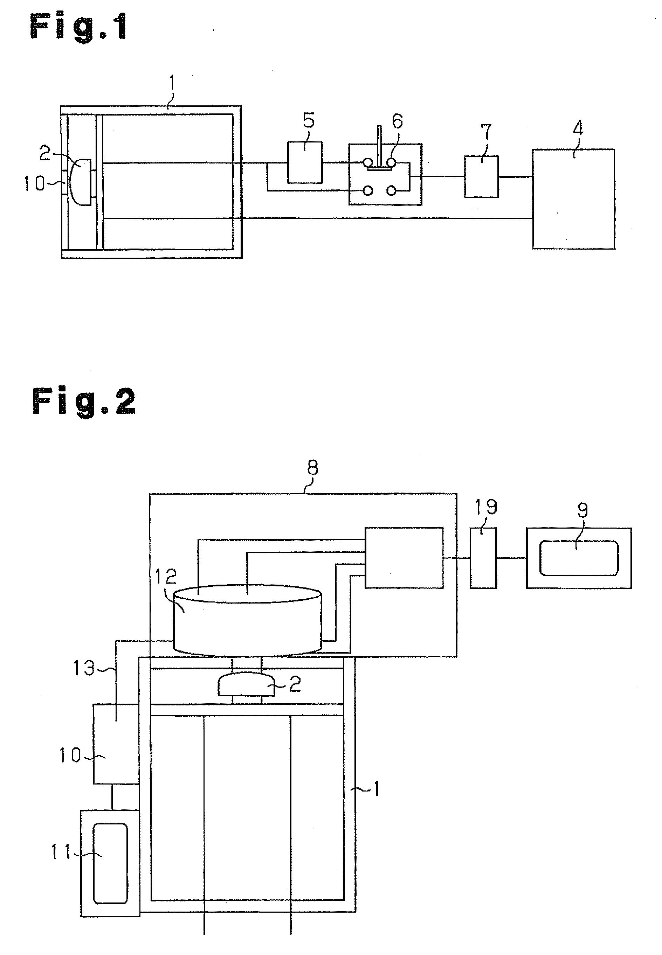

The present invention will now be discussed with reference to the embodiments shown in the accompanying drawings. Fig. 1 shows the main part of a cell activity reduction apparatus according to the present invention. A cylindrical housing 1 having a diameter of approximately 20 mm accommodates an electromagnetic wave emission unit 2. Electromagnetic radio waves are output to the exterior from the electromagnetic wave emission unit 2 through an opening 10 arranged in the housing 1 and having a diameter of about 2 mm.

A light emitting diode that emits electromagnetic radio waves (millimeter waves) of 50 GHz is used as the electromagnetic wave emission unit 2. However, the electromagnetic wave emission unit 2 is not limited to this frequency as long as electromagnetic radio waves in the range of millimeter waves to terahertz waves of 30 GHz to 3 THz can be emitted. Further, the electromagnetic wave emission unit 2 is not limited to the light emitting diode as long as electromagnetic waves in the above wavelength range can be emitted.

The electromagnetic wave emission unit 2 emits the electromagnetic waves at an intensity of approximately 1 µW. However, the emission intensity is not limited to this value and may be changed to an appropriate intensity obtained through experiments of the like.

A switch 6 is used to switch between pulse illumination, which is performed by a pulse illumination controller 5, and continuous illumination, which is performed by directly connecting a power supply 4 and the electromagnetic wave emission unit 2. Further, a timer 7 controls the emission time.

Fig. 2 shows an experiment conducted to prove the cell activity reduction effect obtained by the electromagnetic wave emission described above. In the drawing, reference character 12 denotes a cell holding unit that holds cells, which is the irradiation subject, together with a culture liquid. The cells are connected to an electric resistance measurement unit 8, which measures the electric resistance of the cells, and an electric resistance value display unit 9, which displays the measured electric resistance value. Further, a pH measurement unit 10, which measures the pH of the culture liquid, and a pH value display unit 11, which displays the measured pH value, are connected. In the drawing, reference character 13 denotes a sensing unit in the pH measurement unit.

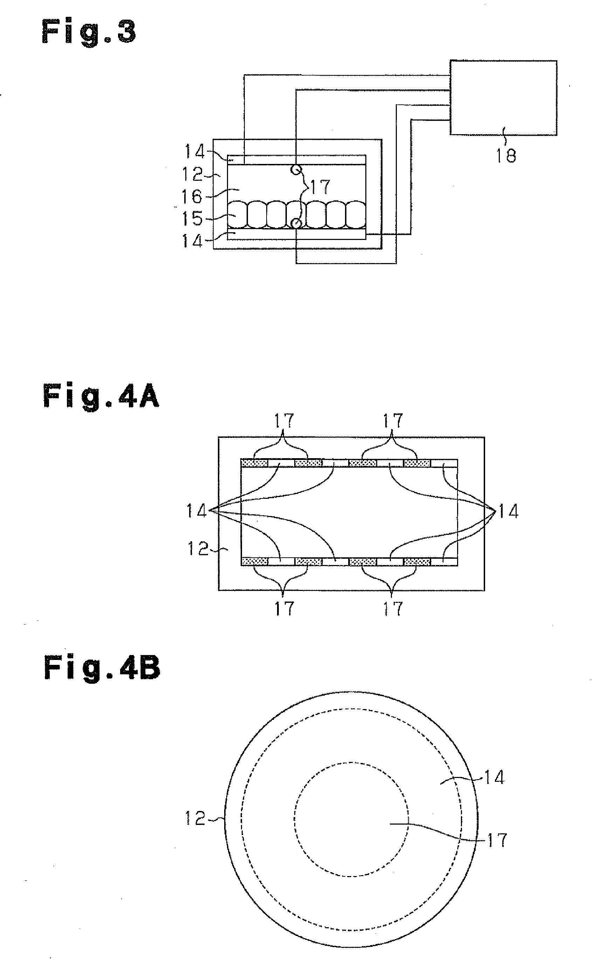

Fig. 3 shows the details of the electric resistance measurement unit 8. Supply electrodes 14 are arranged on two opposing inner surfaces of the cell holding unit 12, which is filled with cells 15 and a culture liquid 16. Constant current is supplied from a power supply 18 to the supply electrodes 14 so that constant current flows in the culture liquid 16, which is electrolytic, and voltage changes in the culture liquid can be detected with a detection electrode 17, which is arranged in the cell holding unit 12. The value of the constant current and the voltage value obtained by the detection electrode 17 are input to a resistance calculation unit 19 to calculate the resistance value in the cell holding unit 12 and show the resistance value on the cell electric resistance value display unit 9.

When the cell activity rises, specifically, when the number of cells in the cell holding unit 12 increases, when the forms of the cells changes such as the cells being enlarged in shape, or when the conditions in the cells become active, the resistance value in the overall cell holding unit 12 increases. On the other hand, when the cell activity falls, specifically, when the number of cells in the cell holding unit 12 decreases, when the forms of the cells changes such as the cells being contracted in shape, or when the conditions in the cells become inactive, the resistance value in the overall cell holding unit 12 decreases. Changes in the cell activity can be checked by measuring the resistance value.

The electric resistance measurement unit 8 is not limited to the structure described above. Any structure may be used as long as the resistance value of the culture liquid can be measured. For example, as shown in Fig. 4A, supply electrodes 14 and detection electrodes 17 may be alternately arranged on two corresponding inner surfaces of the cell holding unit 12. Alternatively, as shown in Fig. 4B, a supply electrode 14 and a detection electrode 17 may be arranged in concentricity on two opposing inner surfaces in the cell holding unit 12.

Although constant current is applied to the supply electrode 14, constant voltage may be applied to the supply electrode 14. In this case, current changes are detected by the detection electrode to calculate the resistance.

Culture liquid is used as a liquid filled in the cell holding unit. However, there are other liquids that allow for measurement within a short period although this depends on the cells. Thus, the liquid is not limited to culture liquid.

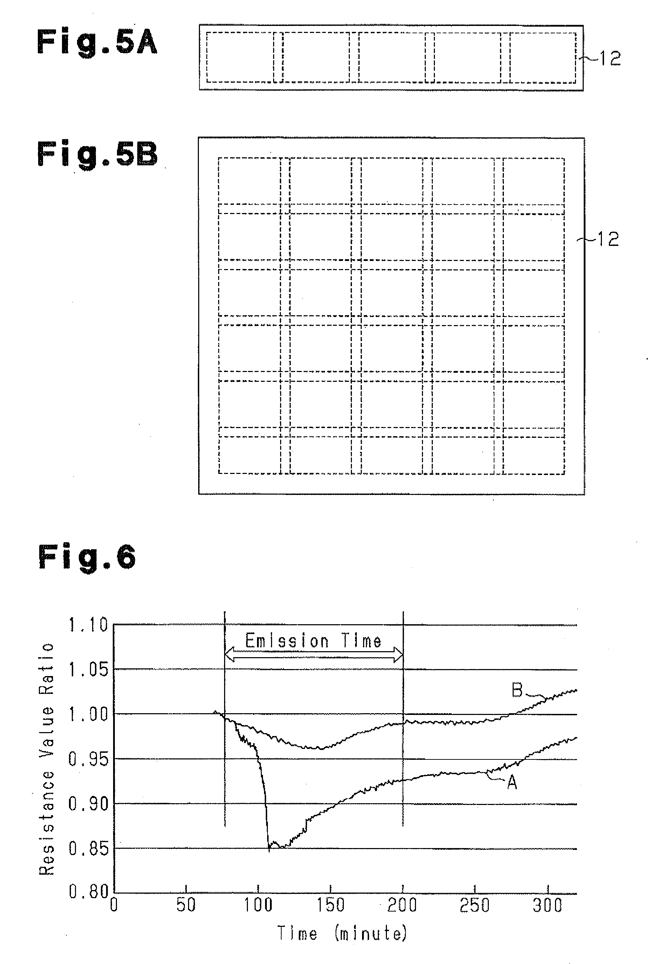

The cell holding unit 12 does not have to include only one compartment. For example, as shown in Fig. 5, the cell holding unit 12 is preferable for use when there is a plurality (thirty in the illustrated example) of compartments. When irradiating a plurality of cells of the same type with electromagnetic waves of the wavelength bands described above or when irradiating cells of the same type to check repeatability, the plurality of compartments of the cell holding unit allows for various observations to be made at the same time and is thus advantageous.

A specific example will now be described. Fibroblasts were used as cells irradiated with electromagnetic waves. The cell holding unit 12 included two compartments. Cells and culture liquid were divided into two and put into the two compartments. The cells in one of the compartments were irradiated with electromagnetic waves and used as specimens of which changes were observed. The cells in the other one of the compartments were used as control specimens.

Until the emission of electromagnetic wave was completed, the cell activity reduction apparatus including the cell holding unit 12 was kept in a CO2 incubator maintained at a temperature of 37°C. a humidity of 100%, and 5% of CO2.

The cell holding unit 12 was left for 70 minutes in the incubator to stabilize the cells in the cell holding unit 12. Then, the resistance value in the cell holding unit 12 was started, and the resistance value was measured for ten minutes. Afterwards, continuation illumination was performed for 120 minutes to emit electromagnetic waves of 50 GHz.

In this state, the intensity of the electromagnetic waves emitted from the electromagnetic wave emission unit 2 was approximately 1 µW. The intensity of the electromagnetic waves when reaching the cells 15 was approximately 1 µW to 1 pW.

Changes of the resistance value in the cell holding unit 12 are shown in Fig. 6. The electromagnetic wave emission corresponds to the period of 80 minutes to 200 minutes in Fig. 6. Then, the emission was stopped for 120 minutes (corresponding to 190 minutes to 320 minutes in Fig. 6) for observation. The resistance value shown in Fig. 6 uses 1 as an initial value and is indicated as a ratio of a relative change amount. Thus, the resistance value has no units, the resistance value ratio is plotted every minute.

As apparent from Fig. 6, a resistance value ratio A of the specimens irradiated with electromagnetic waves and a resistance value ratio B of the control specimens that were not irradiated with electromagnetic waves shifted in the same manner until the electromagnetic waves were emitted. However, after five minutes from the emission of electromagnetic waves, the resistance suddenly started to decrease in only the irradiated cells. The resistance continued to decrease over approximately the next 20 to 25 minutes. Then, over approximately 20 minutes, the resistance value remained decreased without any substantial changes. Subsequently, the resistance gradually increased but stopped increasing after approximately 15 minutes. Then, the resistance ratio A of the irradiated cells shifted as it changed in the same manner as the control cells while maintaining a lower resistance value than specific resistance value B of the control cells.

This confirmed that the electromagnetic wave emission reduced the cell activity. This change is not a sudden change and is a change in the resistance value that occurs over time. It is thus apparent that the change is not a result of noise or the like.

In the present embodiment, the resistance value ratio is used to check the cell activity. However, the pH value of the culture liquid 16 may be used to check the cell activity. Specifically, when the pH value of the culture liquid 16 becomes high, it may be determined that the cell activity is low.

When the electromagnetic wave emission decreases the resistance value ratio of specimens, that is, when the cell activity decreases, there is a tendency for the pH value of the culture liquid 16 to increase.

When the electromagnetic wave emission unit 2 emitted electromagnetic waves of different frequencies in the range of 30 GHz to 3 THz, particularly, 30 to 80 GHz, although there was a slight difference in level, substantially the same results were obtained. When emitting electromagnetic waves of wavelengths longer than 30 GHz and wavelengths shorter than 3THz, significant changes in the resistance value could not be observed.

Significant decreases in the resistance value ratio were also observed for pulse illumination.

In the present embodiment, changes in the cell activity resulting from the electromagnetic wave emission are determined from the resistance value ratio of the cells or the pH value of the culture liquid. However, the determination of changes in the cell activity is not limited in such a manner. More specifically, as long as changes in the activity of the specimens that are the irradiation subject of the electromagnetic waves may be observed over time, any scheme may be employed. For example, an X-ray image may be used to visually check the specimens, changes may be measured using activation markers by sampling some of the specimens, and factors corresponding to cell activation and released out of the cells may be measured.

## Claims

1. A cell activity reducing method being **characterized by**:
emitting electromagnetic waves having a frequency of 30 GHz to 3 THz to cells to reduce activity of the cells.

2. A cell activity reduction apparatus being **characterized by**:
an electromagnetic wave emission means for emitting electromagnetic waves having a frequency of 30 GHz to 3 THz that reduces cell activity.

3. The cell activity reduction apparatus according to claim 2, being **characterized by**:
an electric resistance measurement means for measuring an electric resistance of the cells, which is an irradiation subject of electromagnetic waves, or a culture liquid for the cells, which is an irradiation subject of electromagnetic waves; and
a display means for displaying a value of the electric resistance measured by the electric resistance measurement means.

4. The cell activity reduction apparatus according to claim 2 or 3, being **characterized by**:
a pH measurement means for measuring the pH of a culture liquid for cells, which is an irradiation subject of electromagnetic waves; and
a display means for displaying a value of the pH measured by the pH measurement means.
